## Europäisches Patentamt
## European Patent Office
## Office européen des brevets

(11) Veröffentlichungsnummer: **0 034 775**
**A2**

(12) **EUROPÄISCHE PATENTANMELDUNG**

(21) Anmeldenummer: **81101026.3**

(22) Anmeldetag: **13.02.81**

(51) Int. Cl.³: **G 01 N 33/54**

(30) Priorität: **22.02.80 DE 3006709**

(43) Veröffentlichungstag der Anmeldung:
**02.09.81 Patentblatt 81/35**

(84) Benannte Vertragsstaaten:
**AT BE CH DE FR GB IT LI LU NL SE**

(71) Anmelder: **Thoma, Hans A., Dr.**
**Giselastrasse 3**
**D-8000 München 40(DE)**

(72) Erfinder: **Thoma, Hans A., Dr.**
**Giselastrasse 3**
**D-8000 München 40(DE)**

(74) Vertreter: **Hansen, Bernd, Dr.rer.nat. et al,**
**Hoffmann . Eitle & Partner Patentanwälte**
**Arabellastrasse 4**
**D-8000 München 81(DE)**

(54) **Homogenes Verfahren zur kompetitiven Bestimmung von Liganden.**

(57) Ein homogenes Verfahren zur kompetitiven Bestimmung von Liganden, insbesondere solcher höhermolekularer Spezifikation, unter Verwendung von Enzymmodulator als Marker des zu bestimmenden Liganden, von Enzymmodulator-Ligand-Konjugat bindendem Rezeptor, Enzym und Substrat wird beschrieben, wobei mindestens einer der die Messreaktion beeinflussenden Reaktionspartner sterisch vergrössert wird.

Fig.1

EP 0 034 775 A2

## Homogenes Verfahren zur kompetitiven Bestimmung von Liganden

Die Erfindung betrifft ein homogenes Verfahren zur kompetitiven Bestimmung von Liganden, insbesondere solche höhermolekularer Spezifikation unter Verwendung von Enzymmodulator als Marker des zu bestimmenden Liganden, von Enzymmodulator-Ligand-Konjugat bindendem Rezeptor, Enzym und Substrat.

Es wurden eine Reihe von Verfahren entwickelt, die als "kompetitive Proteinbindungstests" oder "Sättigungsanalyse" bezeichnet werden. Diese Bestimmungstechniken basieren auf der Fähigkeit eines Rezeptors, im allgemeinen eines Antikörpers, mit einem markierten Liganden einen Komplex zu bilden, und zwar mit einer hohen molekularen Spezifität. Während die Spezifität dieser Techniken im wesentlichen auf der Schlüssel-Loch Komplementarität beruht, ist die Sensitivität abhängig von der Komplexbildungskonstante (thermodynamische Gleichgewichtskonstante) K von Rezeptor (Ak) und Ligand. Bei Verwendung eines markierten Liganden und einer Konkurrenzreaktion von markierten und unmarkierten Liganden um die Bindungsstellen ist die Menge des gebundenen markierten (bei konstanter Konzentration) Liganden eine Funktion der Konzentration des unmarkierten Liganden.

- 2 -

Dazu ist erforderlich, dass entweder

(a)     freier und antikörpergebundener markierter Ligand durch geeignete Verfahren getrennt werden/ heterogenes Verfahren wie z.B. RIA oder ELISA oder

(b)     freier und antikörpergebundener markierter Ligand unterschiedliche Signale geben.

Als Markierung für die Liganden sind eine Reihe von Stoffen mit speziellen physikochemischen oder biochemischen Eigenschaften beschrieben worden.

Für diese homogenen Verfahren sind eine Reihe von Techniken entwickelt worden, die eine enzymatische Reaktion als Signal benützen, z.B. die DE-AS 22 23 385. Diese und ähnliche Verfahren arbeiten mit Konjugaten von Liganden mit einem Reaktionsteil aus einer enzymatischen Reaktion, z.B. Ligand-Enzym, Ligand-Coenzym, Ligand-Enzymmodulator, etc.. Durch die Komplexbildung des Ligandenanteils mit dem Rezeptor wird der Teil des Konjugates, der in die enzymatische Reaktion eingeht, in seinem Reaktionsverhalten geändert und ergibt dadurch eine indirekte Messgrösse für den rezeptorgebundenen oder freien Ligandenanteil des Konjugates.

Da diese Effekte fast ausschliesslich auf sterischen Wechselwirkungen der Konjugate nach der Komplexierung mit dem Rezeptor basieren, entstehen dadurch auch Limitierungen für die Liganden, bei denen diese Techniken angewandt werden können. Denn dieses Prinzip funktioniert meistens nur solange, wie das Ligandmolekül räumlich nicht allzu gross ist, d.h. solange ein enger räumlicher Kontakt zwischen Rezeptor (Antikörper) und Marker möglich ist. Bei einem grossen Ligandmolekül, z.B. einem Protein, ist die Wechselwirkung zwischen Ligand und Antikörper zu weit vom gekoppelten Marker entfernt, um dessen Aktivität beeinflussen zu können. Somit sind die meisten dieser homogenen Enzymmarkierungstechniken auf die Bestimmung von niedermolekularen Substanzen beschränkt.

Der Erfindung liegt die Aufgabe zugrunde, ein Verfahren zur kompetitiven Bestimmung von Liganden zu schaffen, das in einfacher Weise auf eine Fülle von Liganden anwendbar ist und insbesondere auch bei der Bestimmung höhermolekularer bzw. grosser Liganden eine hohe Erfassungsgrenze bei gleichzeitig sehr guter Genauigkeit aufweist. Insbesondere soll es hierdurch

- 4 -

möglich sein, die Liganden durch homogene Methodiken, d.h. ohne notwendige Trenntechniken, quantitativ bestimmen zu können.

Diese Aufgabe wird durch die Schaffung eines Verfahrens der eingangs genannten Art gelöst, das dadurch gekennzeichnet ist, dass mindestens einer der die Messreaktion beeinflussenden Reaktionspartner sterisch vergrössert wird.

Durch die sterische Vergrösserung zumindest eines der Reaktionspartner ist es möglich, die spezifischen Bindungsverfahren zum Nachweis und zur Bestimmung von Liganden gemäss der DE-PS 27 54 086 (Miles Laboratories, USA) auf die Bestimmung grösserer oder höhermolekularer Liganden auszuweiten und insbesondere die Messgenauigkeit solcher Verfahren zu erhöhen. Durch den Einsatz der dort beschriebenen reversibel bindenden Enzymmodulatoren und insbesondere solchen mit einer Bindungskonstanten für die reversible Bindung zwischen Enzymmodulator und Enzym zwischen $10^5$ bis $\sim 10^9$ $m^{-1}$ ist die Anwendung der herkömmlichen Verfahren durch eine zum Teil unzureichende Messgenauigkeit behindert, zum Teil aber auch auf die Bestimmung grösserer Liganden wegen deren Grösse nicht mehr anwendbar. Das erfindungsgemässe Verfahren kann jedoch mit besonderem Vorteil bei Verfahren zur Bestimmung von Liganden angewandt werden, bei denen Enzymmodulatoren, die das Bezugsenzym irreversibel und/oder kovalent binden, eingesetzt werden. Verfahren unter Anwendung derartiger Enzymmodulatoren als Marker sind in der am gleichen Tage eingereichten deutschen Patentanmeldung P 30 06 710.8 des Anmelders (Titel: Enzymmodulator-Ligand-Konjugat und Verfahren unter dessen Anwendung zur Bestimmung von Liganden und insbesondere biologisch wirksamen Verbindungen) beschrieben worden, worauf ausdrücklich Bezug genommen
wird. Die dort beschriebenen Enzymmodulatoren können
insbesondere sogenannte "Affinity labeling"-Substanzen
bzw. "Active site directed"-Reagentien, aber auch Haloketonanaloge von NAD, site-spezifische Haloketone, Haloacetylderivate oder Halomethylverbindungen darstellen. Darüber
hinaus finden mikrobiell erzeugbare Inhibitoren geringen Molekulargewichtes, wie z.B. Pepstatin, Chymostatin,
etc., wie ausführlich in der Parallelanmeldung des Anmelders beschrieben, eine bevorzugte Anwendung.

Mit der Bezeichnung "Ligand" werden hier grundsätzlich
all diejenigen Liganden erfasst, die in der DE-AS 22 23 385
in den Spalten 22 bis 46 beschrieben worden sind, worauf
ausdrücklich für die Zwecke der vorliegenden Erfindung
Bezug genommen wird. Die kovalente Bindung des Enzymmodulators an den Liganden erfolgt dabei im allgemeinen
an den Stellen des Liganden, wie dies ebenfalls in der
DE-AS 22 23 385 beschrieben worden ist. Lediglich zur Erläuterung sei hier kurz angeführt, dass es sich hierbei unter
anderem um Antigene, um Haptene oder auch um natürlich vorkommende Rezeptoren handeln kann. Unter diese Gruppen fallen
eine Fülle biologisch aktiver Systeme, wie Hormone, z.B.
Thyroxin, viele Steroide, wie die Östrogene, Kortison,
Kortikosteron und Östrodiol, Polypeptide, wie Insulin, aber
auch Wirkstoffe von Arzneimitteln als solche, wie z.B. Prostaglandine, Zucker, häufig eingenommene Drogen, wie Benzdiazepinone, Antibiotika, etc.

Bei der Bezeichnung "Rezeptor" handelt es sich im Regelfall um sogenannte Antikörper, deren Gewinnung beispielsweise durch Immunisierung von Tieren mit entsprechend hoch gereinigten Antigenen, Carrier-gebundenem Hapten,
etc. bekannt ist. Die Antikörper sind häufig $\gamma$-Globu-
line, die, wie eingangs angeführt, mit hoher Spezifität den Liganden zu binden vermögen. ·

Mit der Bezeichung "Enzym" werden solche Enzyme bezeichnet, die sich in einer Messreaktion mit der Enzymmodulatorkomponente des Enzymmodulator-Ligand-Konjugates reversibel, ·
oder bevorzugt, praktisch irreversibel und kovalent binden,
wodurch sich eine dementsprechend quantitative "Störung"
bzw. "Blockierung" der Enzymaktivität ergibt. Die (Bezugs)-
Enzyme können somit aus der Reihe der auf dem Gebiet der
Enzymologie bekannten bzw. den in der obigen Parallelanmeldung des Anmelders angeführten Enzymen,in Abstimmung
mit der gewählten Enzymmodulatorsubstanz des Konjugatkomplexes ausgewählt werden. Diese können im Hinblick auf
die Kriterien der Billigkeit, der Spezifität der Wirkung,
ihrer Stabilität, ihrer Nachweisbarkeit, ihres Nichtvorliegens im endogenen System, etc., gewählt werden, wobei
lediglich illustrativ z.B. D.H.F.-Reduktase, Pepsin, vorteilhaft auch in derivatisierter Form, z.B. als Acetylverbindungen, Chymotrypsin, Esterase, Leucin, Aminopepsidase,
Cathepsin D, Proteinase, etc., genannt werden können.

Das Enzymmodulator-Ligand-Konjugat wird im allgemeinen
aus dem quantitativ, kovalent und irreversibel bindenden oder nicht-kovalent reversibel bindenden Enzymmodulator, wobei letzere Alternative weniger bevorzugt ist, und
dem Ligand nach Methodiken analog erzeugt, die von der Bildung von Ligand-Marker-Konjugaten, bei denen andere Markersubstanzen, wie z.B. Enzyme, Coenzyme, etc., Verwendung finden, grundsätzlich bekannt sind. Die Kopplungsreaktionen hängen im wesentlichen von den am Ligand bzw. Enzymmodulator zur Verfügung stehenden bzw. einführbaren reaktiven bzw. aktivierbaren Gruppen ab. Im allgemeinen werden die bekannten Methoden der Peptid-Chemie, der Affinitätschromatografie, der Solid-Phase-Technologie, ganz analog angewandt. Mit Vorteil kann man, z.B. bei kleinen Haptenen, analoge Reaktionen, die zur Synthese des Antigens, d.h. Kopplung an Albumin, Thyroglobulin etc., verwendet werden, einsetzen. Vorteilhaft sind auch Kopplungsverfahren mit bifunktionellen Reagentien, wie z.B. Carbodiimiden (z.B. 1-Cyclohexyl-3-(morpholinäthyl)-carbodiimid), Glutaraldehyd, m-Maleinimidobenzyl-N-hydroxysuccinimidester, etc., sowie spezielle Verfahren, wie die reduktive Aminierung, gemischte Anhydridmethoden, Thioätherverfahren, etc. Bei grösseren Liganden ist manchmal auch die Einführung einer verlängerten Kette, auch "Spacer" oder "Brücke" genannt, mit einer Länge von etwa 1 bis 20 C-Atomen vorteilhaft.

Das erfindungsgemässe Verfahren beruht grundsätzlich ebenfalls darauf, dass ein Konjugat, bestehend aus Ligand, gebunden an einen Enzymmodulator, mit dem gegen den Ligand gerichteten Rezeptor einen Komplex eingehen kann und somit mit nicht-markiertem (konjugiertem) Ligand um die Bindungsstellen am Rezeptor konkurriert.

Die Bildung dieses Komplexes kann, je nach Art des zu bestimmenden Liganden, häufig zunächst noch nicht oder nur zu einem geringen Anteil die Modulatorwirkung mit dem korrespondierenden

- 8 -

Enzym verändern. Erst durch die erfindungsgemäss erfolgende sterische Vergrösserung mindestens eines, der die Messreaktion beeinflussenden Reaktionspartner werden die Bedingungen geschaffen, dass die Behinderung einer Annäherung des Enzyms an den Modulatoranteil des komplexierten Ligand-Enzymmodulator-Konjugates in ausreichendem Masse gewährleistet ist.

Erfindungsgemäss werden mit Vorteil die Vergrösserung des Rezeptors, des Enzyms, eine Kombination von vergrössertem Rezeptor und vergrössertem Enzym oder auch ein vergrösserter Enzymmodulator,bzw. entsprechende Kombinationen hiervon in Betracht gezogen.

Die Vergrösserung der genannten Reaktionspartner kann mit Vorteil durch den Einsatz von Antikörpern oder deren entsprechenden Fab-Teilen erfolgen.

Die sterische Vergrösserung des Rezeptors, d.h. eines Ligand-Antikörpers, kann durch Zugabe eines Anti-Antikörpers und die Bildung eines daraus resultierenden Komplexes Antikörper-anti-Antikörper erfolgen. Durch die sterische Vergrösserung des Rezeptors wird auch bei grossen Antigenen als Ligand die Annäherung des Enzyms an den Modulatoranteil des komplexierten Ligand-Enzymmodulator-Konjugates im ausreichenden Masse verhindert.

Die erfindungsgemäss zusätzlich vorgesehene Behinderung der Reaktion Enzym mit der komplexierten Modulatorkomponente des gebundenen Ligand-Enzymmodulator-Konjugates kann auch auf andere Weise erreicht werden, z.B. durch Vergrösserung des Bezugsenzyms, so dass das Enzym von sich aus an der Annäherung an das Konjugat sterisch gehindert ist. Die Vergrösserung des Enzyms kann durch Reaktion des Enzyms mit Anti-Enzym-Antikörper erfolgen, die nicht gegen das aktive Zentrum gerichtet ist. In analoger Weise kann eine Vergrösserung des Enzyms durch chemische Fixierung des Enzyms an wasserlösliche Polymere, wie z.B. Polylysine oder Dextrane, etc., durchgeführt werden.

Bei der Vergrösserung des Enzyms bzw. der Rezeptorkomponente können anstelle der Antikörper, z.B. zur Vermeidung von Präzipitationen, erfindungsgemäss auch deren Fab-Teile eingesetzt werden.

Bei anderen Anwendungen des erfindungsgemässen Verfahrens kommt auch der kombinierte Einsatz einer sowohl vergrösserten Rezeptorkomponente, z.B. durch die Zugabe von gegen den spezifischen antigenbindenden Rezeptor wirkenden Antikörpern, mit Anti-Enzym-Antikörpern bzw. Fab-Teilchen-vergrössertem Enzym oder Enzymen, die an lösliche Polymere gebunden sind, in Betracht.

Stellt der Ligand z.B. ein sehr grosses Antigen dar, z.B. Australia-Antigen, während das Bezugsenzym relativ klein gewählt ist, kann erfindungsgemäss auch unter Zuhilfenahme von Antimodulator-Antikörpern folgendes Verfahren eingesetzt werden:

Der Komplexbildung von Ligand- bzw. Antigen-Enzymmodulator-Konjugat mit dem spezifisch bindenden Rezeptor (Antikörper) der eventuell auch zusätzlich schon mit Anti-Antikörper-Komplexen vergrössert ist, folgt die Zugabe von Anti-Enzymmodulator-Antikörpern, eventuell ebenso mit Anti-Antikörpern einer anderen Tierspezies oder durch Bindung an wasserlösliche Polymere vergrössert. Diese vergrösserten Antikörper gegen die Enzymmodulatoren werden bei diesem Verfahren an der Annäherung an das Antikörper-komplexierten Ligand-Modulator-Konjugat gehindert, sind aber in der Lage, die Enzymmodulatoranteile der nicht-komplexierten Antigenmodulator-Konjugate zu binden. Eine nun erfolgende Enzymzugabe bewirkt, dass das kleine Enzym nun nur noch durch die gebundenen Enzymmodulator-Anteile der Antigen-Modulator-Konjugate beeinflusst wird.

Während somit in den erstgenannten Fällen die Messgrösse direkt das freie Enzymmodulator-Antigen-Konjugat darstellt, da die Modulatoreigenschaften noch unverändert sind, wird im zuletzt beschriebenen Fall in den Messreaktionen der gebundene Anteil des Enzymmodulatoranteils im Konjugat erfasst.

Die Durchführung des erfindungsgemässen Verfahrens kann derart erfolgen, dass in eine wässrige, gegebenenfalls gepufferte Lösung, die Probe, der spezifisch bindende Rezeptor (Antikörper), der Anti-Antikörper einer anderen Tierspezies, das Antigen-(Hapten)Modulator-Konjugat, in speziellen Fällen der Antimodulator-Antikörper, der Anti-Antimodulator-Antikörper, das Enzym, eventuell an wasserlösliche Polymere gebunden, manchmal der Anti-Enzym-Antikörper und das Substrat zusammengebracht werden und die Geschwindigkeit der Enzymreaktion gemessen wird.

Die Reihenfolge der Zugabe der einzelnen Komponenten hängt von der zu bestimmenden Substanz ab, wobei im erfindungsgemässen Verfahren, wie bereits eingangs dargelegt, es manchmal ausreichend ist, nur einen der die Messreaktion beeinflussenden Reaktionspartner sterisch zu vergrössern. Allerdings kann dies, sofern gewünscht, auch bei mehreren der Reaktionspartner, z.B. sowohl beim Enzym wie beim gebundenen Enzymmodulator, etc., erfolgen.

Die Reihenfolge der Zugabe der einzelnen Komponenten hängt von dem zu bestimmenden Liganden ab, worauf im folgenden noch näher eingegangen wird. Unabhängig von der gewählten Reihenfolge der Zugabe der jeweiligen Komponenten kann es erfindungsgemäss im allgemeinen bevorzugt sein, die zu der gewünschten sterischen Vergrösserung des bzw. der Reaktionspartner erfolgende Zugabe der Antikörper vor Zugabe dieses Reaktionspartners zu dem Reaktionsgemisch durchzuführen bzw. mit Antikörper zur sterischen Vergrösserung vorreagierte Komponenten einzusetzen.
Die Herstellung der in der Messreaktion eingesetzten Komponenten erfolgt in an sich bekannter Weise. Bezüglich der Herstellung von Ligand-Enzymmodulator-Komplexen, soweit der Enzymmodulator reversibel ist, wird auf die DE-AS 27 54 086 (Miles) verwiesen. Bezüglich des Einsatzes von irreversibel, kovalent bindenden Enzymmodulatoren kommen Verfahren nach der vorstehend genannten parallelen Anmeldung des Anmelders vom gleichen Tage in Betracht, wobei zusätzlich noch für die jeweiligen Bindungsstellen der Liganden allgemein auf die DE-AS 22 23 385 (Syva) verwiesen wird.

Die Gewinnung der jeweiligen Antikörper sowie der entsprechenden Fab-Fragmente ist für sich bekannt. Im Rahmen des erfindungsgemässen Verfahrens ist es dabei auch möglich, als gewünschte Antikörper solche unterschiedlichen tierischen Ursprungs in Kombination einzusetzen. So können z.B. Antikörper, die gegen den Liganden wirken, beispielsweise als Kombination von Antikörper von Kaninchen und von Antikörpern von Schafen zugeführt werden, etc. Auch hierdurch kann eine bessere sterische Abdeckung erreicht werden.

Die Verfahren unter Anwendung des Enzymmodulator-Ligand-Konjugates zur Bestimmung eines Liganden und insbesondere biologisch wirksamer Verbindungen, zeichnen sich z.B. dadurch aus, dass man in einem flüssigen Medium die (Serum)Probe, das Enzymmodulator-Ligand-Konjugat, einen unter Beeinflussung der Aktivität der Enzymmodulatorkomponente reagierenden Rezeptor, das Bezugsenzym und ein Substrat hierfür in geeigneter Reihefolge zusammenführt und die Aktivität des Bezugsenzyms misst. Hierbei sind eine oder gegebenenfalls mehrere der vorstehend genannten Komponenten im Regelfall durch vorherige Reaktion bereits sterisch vergrössert. Das Verfahren kann in homogener Weise durchgeführt werden, was häufig die bequemste Methodik darstellt.

Genauso ist es aber auch möglich, eine heterogene Verfahrensweise anzuwenden, wobei die bekannten Trenntechniken, wie Solid-phase-Technik (immobilisierter Rezeptor), Trennungstechniken, wie z.B. mit Ammoniumsulfat, Polyäthylenglykol (PEG) oder Doppelantikörper und Adsorptionstechniken, wie z.B. an Kohle, Ionenaustauscher etc., zugrundegelegt werden. Nach erfolgter Trennung wird die entsprechend verbliebene Modulatorkonjugataktivität
(frei oder gebunden) vermessen.

Die homogene Arbeitsweise beruht im Prinzip darauf,
dass nach der Komplexierung des Ligand-Enzymmodulator-
Konjugates mit dem Rezeptor, normalerweise einem Antikörper, die Enzymmodulatorwirkung verloren geht bzw.
stark verändert wird. Durch eine anschliessende Reaktion des Bezugsenzyms mit dem vorhandenen Substrat bzw.
die Inhibierung dieser Reaktion und die Messung
der Enzymaktivität nach bekannten Methoden, vorzugsweise z.B. mit chromogenen oder fluorogenen Substraten oder auch mit gekoppelten Lumineszenzreaktionen,
kann die verbliebene Enzymmodulatoraktivität nachgewiesen
werden.

Die Reihenfolge der Zugabe der einzelnen Komponenten
hängt von der zu bestimmenden Substanz ( = Ligand) ab,
wobei die sich ergebenden verschiedenen Möglichkeiten
nachfolgend noch ausführlicher dargelegt werden. Im
wesentlichen handelt es sich meistens um mehrere Inkubationsperioden hintereinander.

Die nach Bildung des "Komplexes" aus Ligand-Enzymmodulator-Konjugat und Rezeptor vorhandene bzw. verbliebene Enzymmodulatorwirkung ist normalerweise ein Mass für das freie (nicht Rezeptor gebundene) Ligand-Enzymmodulator-Konjugat. Durch Herstellung von Standards mit bekannten Mengen der zu bestimmenden Substanz können Eichkurven erhalten werden, mit denen die zu bestimmenden (im Hinblick auf den Gehalt an Ligand unbekannten) Proben in Beziehung gesetzt werden können. Das gleiche Prinzip lässt sich auch in Form von Solid-phase-Techniken, z.B. mit immobilisierten Antikörpern, durchführen.

Der Nachweis der noch vorhandenen Enzymmodulator- bzw. Inhibitoraktivitäten erfolgt mit Hilfe von Enzymmodulator- bzw. Enzyminhibitorkonzentration abhängigen Bezugsenzymsubstratreaktionen. Bereits vorstehend wurde dargelegt, dass zur Messung dann chromogene oder fluorogene Substrate oder Lumineszenzreaktionen als Hilfsreaktionen verwendet werden.

Bei der Bestimmung von Proteinen kann es bei Herstellung des Ligand-Enzymmodulator-Konjugates, in dem der Ligand demnach ein Protein ist, zweckmässig sein, mehrere Enzymmodulatoren bzw. Inhibitoren pro Molekül anzukoppeln. Die (sterische) Hinderung der Modulatorwirkung kann in diesem Fall nach dem Zutritt von Antikörpern durch zusätzliche Anti-IgG-Antikörper, d.h. eine zusätzliche sterische Hinderung für die Reaktion Enzym/Enzymmodulator, verstärkt werden. Das nicht komplexierte Ligand-Enzymmodulator-Konjugat ist auch hier üblicherweise für das Bezugsenzym zugänglich, weshalb die gemessene Aktivitätsveränderung des Enzyms mit dem freien, d.h. nicht antikörpergebundenen Anteil des Ligand-Enzymmodulator-Konjugates korrespondiert.

Wenn das Enzym, wie beispielsweise im Falle von Pepsin, relativ klein ist, der nachzuweisende Ligand aber beispielsweise sehr gross ist, z.B. das "Australia"-Hepatitis-Antigen, so ist der gekoppelte Enzymmodulatoranteil, auf den nachstehend auch als Inhibitoranteil Bezug genommen wird, sterisch eventuell schwer zu blockieren. In diesem Fall kann durch den Einsatz von Anti-Inhibitor-Antikörpern zusammen mit Anti-IgG-Antikörpern (möglicherweise schon vorreagiert), die Inhibitorwirkung des gebundenen (im Antikörper-Antigen-Komplex gebundenen) Modulators mit anschliessender enzymatischer Indikatorreaktion erfasst werden.

Werden in diesem Falle sogenannte Solid-phase-Techniken mit immobilisierten Antikörpern angewandt, so kann die Messgrösse sowohl das freie als auch das gebundene Ligand-Enzymmodulator-Konjugat, je nach Durchführung der Methodik, erfassen.

Es braucht hier nicht weiter dargelegt werden, dass im Hinblick auf die Enzymreaktion vorliegenden Substrate besonders ausgewählt werden. Hier sind spezielle "massgeschneiderte" chromogene oder fluorogene Substrate, z.B. für kleine Peptide z.B. proteolytische Enzyme, bekannt, die nach Reaktion eine starke Änderung der Absorption, Extinktion Emission, des Emissionsmaximums, etc., ergeben. Hierbei ist es bekannt, auch die Empfindlichkeiten durch Kopplung mit einem Lumineszenzenzymsystem zu steigern. Wenn eine heterogene Durchführung der Reaktion gewählt wird, bieten sich bekannte Solid-phase-Substrate an, z.B. Proteine (Kaseine oder Hämoglobin) oder spezielle kleinere Peptide, die an eine feste Matrix gekoppelt sind, z.B. an eine Gelfiltrationsmatrix, Polyacrylamid, Glaspartikel, Agarose, etc.. Zur Optimierung sind auch Kopplungen an Enzymketten möglich.

Es gibt eine Vielzahl von Möglichkeiten, das grundsätzliche, erfindungsgemässe Verfahren auszuführen. Dies soll nachstehend weiter erörtert werden:

Das Verfahren zur Messung des Liganden, d.h. Haptens, oder auch Antigens in einer biologischen Probe kann sich z.B. durch folgende Schritte auszeichnen:

a) Der Probe wird eine bestimmte Menge (Überschuss) eines Antikörpers zugesetzt, der gegenüber dem Ligand in der Probe spezifisch ist, um einen Ligand-Antikörper-Komplex zu bilden.

b) Dem unter a) gebildeten Gemisch wird eine bekannte Menge einer Lösung des Ligand-Enzymmodulator-Konjugates zugesetzt und zwar möglichst mit einem Überschuss gegenüber der Menge, die erforderlich ist, um die Menge des nicht gebundenen Rezeptors bzw. Antikörpers zu komplexieren.

c) In dem Gemisch von b) erfolgt die Zugabe einer bekannten Menge einer Lösung des für den Enzymmodulator-Teil spezifischen Enzyms, so dass der nicht rezeptorgebundene Anteil möglichst quantitativ an das Enzym gebunden wird und modulatorisch, d.h. im allgemeinen inhibitorisch, wirkt.

d) Zu dem Gemisch c) erfolgt die Zugabe einer bekannten Menge einer Substratlösung, so dass die verbleibende Enzymkonzentration (Aktivität) erfasst werden kann, z.B. durch ein Substrat mit chromophorer, fluorophorer oder Biolumineszenz-Nachweismöglichkeit. Hierbei sollte die Substratkonzentration derart gewählt sein, dass möglichst eine Enzymsättigung gewährleistet ist.

Das obige Verfahren kann in der angegebenen Reihenfolge, jedoch auch in andersartiger Anordnung, durchgeführt werden. Bei obiger Darstellung ist die vorherige sterische Vergrösserung eines oder mehrerer der Komponenten nicht besonders angeführt worden, um die Darlegung des prinzipiellen Ablaufs der Messreaktion für sich nicht zu komplizieren. Einige der Möglichkeiten sollen nachstehend schematisch anhand von Kurzbezeichnungen der jeweiligen Komponenten dargelegt werden. Hierbei bedeuten

Pr       =    Probe

Ak       =    Rezeptor (=Antikörper gegen Ligand)

Ak(IgG)  =    Antikörper gegen Antikörper (gegen Ligand)

Ak(I)    =    Antikörper gegen Enzymmodulator

Ak(E)    =    Antikörper gegen Enzym

H        =    Ligand (Hapten)

I        =    Enzymmodulator (häufig = Inhibitor)

HI       =    Ligand-Enzymmodulator-Konjugat

E        =    Enzym

S        =    Substrat

/-Ak     =    Rezeptor (=Antikörper) immobilisiert

Ag       =    Ligand (=Antigen)

AgI      =    Antigen-Enzymmodulator-Konjugat

IgG      =    Immunoglobulin

IAk      =    Antikörper-Enzymmodulator-Konjugat

$\overline{Ak}$  =    vorreagierter Antikörper mit Anti-Antikörper oder durch wasserlösliche Polymere vergrössert

Im folgenden werden nun einige Verfahrensvarianten, die nicht als Beschränkung aufzufassen sind, dargestellt:

(1)    a) Pr +/Āk_/
       b) + HI
       c) + E
       d) + S

(2)    a) Pr +/Āk_/+ S
       b) + HI
       c) + E

(3)    a) Pr +/Āk_/+ E
       b) + HI
       c) + S

(4)    a) Pr +/Āk_/
       b) HI + S
       c) + E

(5)    a) Pr +/Āk_/+ E
       b) + HI + S

(6)    a) Pr +/_/-Ak_/+ HI
       b) im Eluat + E
       c) + S

(7)    a) /Āk_/+ HS
       b) + Pr
       c) + E
       d) + S

(8)    a) /Āk_/+ Pr + HI
       b) + E
       c) + S

Die obigen Verfahrensvarianten sind z.B. anwendbar für $T_3$, $T_4$, $rT_3$, $T_2$, etc. ..., für Steroide, wie z.B. Cortisol, Östriol, Östradiol, Testosteron, Progesteron, ... , für Pharmaka, wie Digoxin, Digitoxin, Phenytoin, Diphenylhydantoin, Morphin, Gentamycin, Amphetamin, Barbiturate, etc., sowie auch für Vitame, Nahrungsmittelschadstoffe, Herbicide, Insekticide, Aflatoxine, etc.. Diese Verfahren sind insbesondere für alle Antigene, wie TSH, HCG, Prolactin, IgG, Hepatitis-Antigen, etc., verwendbar.

Das erfindungsgemässe Verfahren wird nachstehend anhand von einigen Versuchsbeispielen zur Herstellung des Konjugates, zur Herstellung von rezeptorgebundenem Konjugat, zur Ermittlung von entsprechenden Eichkurven, u.a. zur Bestimmung der Antikörpermenge erläutert. Hierbei können die Versuchsbedingungen zur Herstellung des Konjugates, zur Reaktion mit Rezeptor etc., in, dem Fachmann bekannter Weise, je nach Wahl des speziellen Systems Ligand-Enzymmodulator-Bezugsenzym, gewählt werden. Im allgemeinen ergeben sich die folgenden Reaktionszeiten für die einzelnen Schritte:

(A) bei Hapten:

| | |
|---|---|
| Reaktionszeit Antikörper/Hapten | 5 min. bis 30 h |
| Reaktionszeit Enzym/Enzymmodulator | 0 min. bis 60 min |
| Enzym-Substratreaktionszeit | 10 sek. bis 60 min |

(B) für grössere Liganden, d.h. Antigene, etc.:

| | |
|---|---|
| Reaktionszeit Antikörper/Antigen | 3 min. bis 30 h |
| Reaktionszeit Enzym/Enzymmodulator | 0 min. bis 60 min. |
| Enzym-Substratreaktionszeit | 10 sek. bis 60 min. |
| Reaktionszeit Rezeptor/Enzymmodulator | 1 min. bis 6 h |

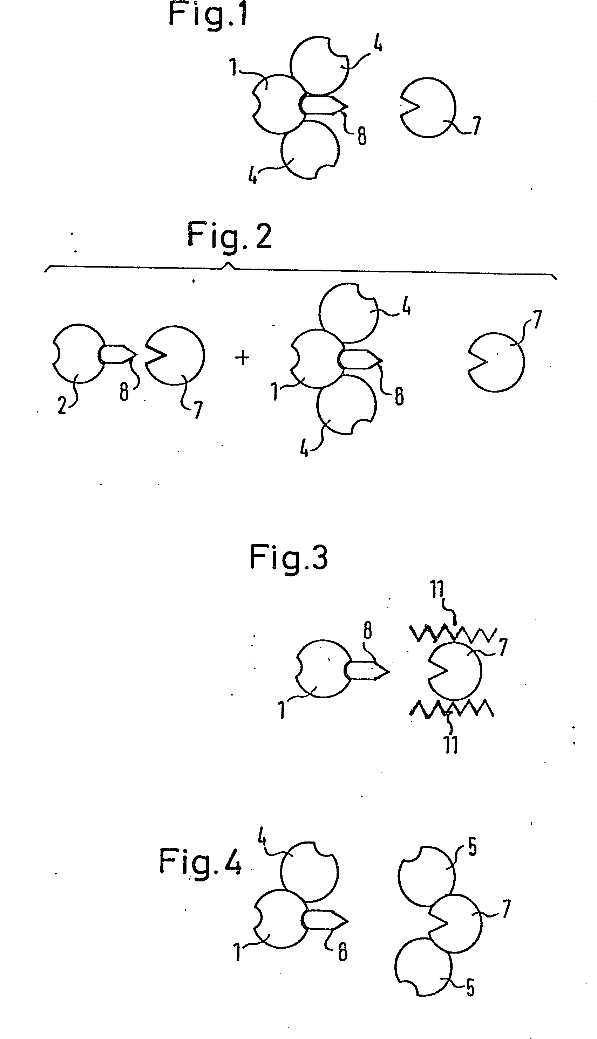

Die erfindungsgemässen Massnahmen sind in den beigefügten Figuren 1 bis 5b schematisch dargestellt. Hierin sind die folgenden Symbole zugrundegelegt:

1 = Antikörper von Kaninchen
2 = Antikörper von Kaninchen gegen Hapten
3 = Antikörper vom Schaf gegen Hapten
4 = Antikörper von der Ziege gegen Antikörper von Kaninchen
5 = Antikörper von Kaninchen gegen Enzym

6 = Antikörper von Kaninchen gegen Inhibitor

7 = Enzym

8 = Hapten-Inhibitor-Konjugat

9 = Antigen

10 = Antigen-Inhibitor-Konjugat

11 = Polylysindextran

= Hapten

= Inhibitor

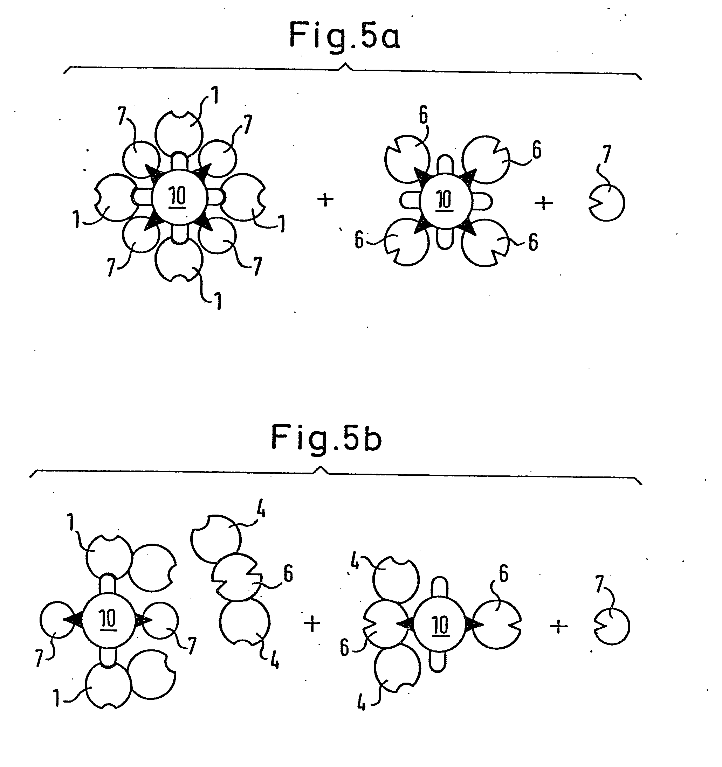

In den Figuren 1, 3 und 4 sind die Messgrössen frei,
während die Figuren 2, 5a und 5b eine gebundene Messgrösse zeigen.

1. Synthese des HuIgG-Pepstatin-Konjugates:

0,1 mMol Pepstatin werden in 10 ml trockenem Dimethylsulfoxid gelöst. Dann werden 10 ml trockenes Tetrahydrofuran
hinzugegeben. Die Lösung wird auf 0°C abgekühlt und mit
0,125 mMol Tri-n-butylamin versetzt. Es folgen 0,1 mMol
Chlorameisensäure-isobutylester. Nach 15 Min. werden
0,1 mMol IgG hinzugegeben. Nach 2 h bei Raumtemperatur
ist die Reaktion beendet. Das Reaktionsgemisch wird mit
Wasser in der Kälte versetzt. Der Niederschlag wird abgesaugt, dialysiert und über einer Sephadexsäule chromatografisch gereinigt.

2. Synthese des Methotrexat-ε-Aminocapronsäure-HuIgG-
   Konjugates:

0,1 mMol Methotrexat werden in 10 ml trockenem Dimethylsulfoxid gelöst. Dann werden 10 ml trockenes Tetrahydrofuran hinzugegeben. Die Lösung wird auf 0°C abgekühlt und
mit 0,125 mMol Tri-n-butylamin versetzt. Es folgen 0,1 mMol
Chlorameisensäureisobutylester. Nach 15 Min. werden 0,1 mMol
ε-Aminocapronsäure hinzugegeben. Unter Rühren erfolgt die
Reaktion innerhalb 2 h bei Raumtemperatur. Das Reaktionsgemisch wurde erneut auf 0°C abgekühlt und mit 0,1 mMol
Tri-n-butylamin versetzt. Es folgen 0,1 mMol Chlorameisensäure-isobutylester. Das Reaktionsgemisch wird nach 15
Min. zu einer Lösung von 150 mg HuIgG in 20 ml Wasser gegeben.
Nach 2 h wird gegen Wasser dialysiert; insgesamt 16 h.
Das Dialysat wird nach chromatografischer Reinigung gefriergetrocknet.

3. Beeinflussung der enzymatischen Aktivität von Pepsin durch HuIgG-Pepstatin-Kunjugat in Anwesenheit von Anti-HuIgG-Antikörper (vom Kaninchen) und Anti-Anti-Ka-JgG-Antikörper (vom Schaf)

Zu 10 µl HuIgG-Pepstatin-Konjugat kommen 10 µl einer Anti-HuIgG-Antikörperlösung (vom Kaninchen) sowie 10 µl einer Anti-Anti-Ka-IgG-Antikörperlösung (vom Schaf), 300 µl Puffer (Citratpuffer pH 5), 200 µl Substratlösung (0,4 mg/ml) (Substrat Phe-Gly-His-4-Nitro-Phe-Phe-Val-ten-OMe).

Reaktionstemepratur: 37°C
Reaktionszeit von IgG-Pepstatin in Anti-HuIgG: 15 Minuten
Reaktionszeit nach Enzymzugabe: 2,5 Min., anschliessend 5 Min. Messzeit.
Reaktionszeit Anti-HuIgG-Antikörper + Anti-Anti-Ka-IgG-Antikörper: 15 Min.

| t (Min.) | Pepsin | + IgG-Pepstatin | + Anti-HuIgG | + Anti-Anti-Ka-IgG |
|---|---|---|---|---|
| | | $E(x10^{-1})$ | | |
| 0,5 | 0,55 | 0,014 | 0,02 | 0,04 |
| 1 | 0,10 | 0,024 | 0,038 | 0,065 |
| 1,5 | 0,145 | 0,03 | 0,055 | 0,088 |
| 2 | 0,184 | 0,036 | 0,058 | 0,106 |
| 2,5 | 0,22 | 0,040 | 0,079 | 0,124 |
| 3 | 0,252 | 0,042 | 0,085 | 0,14 |
| 3,5 | 0,285 | 0,044 | 0,09 | 0,155 |
| 4 | 0,315 | 0,048 | 0,095 | 0,17 |
| 4,5 | 0,34 | 0,05 | 0,1 | 0,185 |
| 5 | 0,364 | 0,05 | 0,105 | 0,2 |
| $\Delta$ 2,5-5 | 0,144 | 0,01 | 0,02 | 0,06 |

Als Ergebnis lässt sich ablesen, dass nach Gleichgewichtseinstellung des Inhibitoranteils des Konjugats mit dem Enzym (ca. nach 2,5 Min.) die enzymatische Aktivität, die durch die Konjugatzugabe fast völlig verschwunden ist ($\Delta$ E 2,5 - 5 Min. = 0,01) durch die Komplexierung des Konjugates mit einem Antikörper zwar etwas vermindert ($\Delta$ E 2,5 - 5 Min.= 0,02), aber eine sterische Blockade durch zusätzliche Anti-Antikörper diese Aktivität des Enzyms (d.h. Inaktivierung des Inhibitoranteils des Konjugates) beträchtlich ($\Delta$ E,2,5 - 5 Min. = 0,06) ansteigen lässt.

Patentansprüche:

1.      Homogenes Verfahren zur kompetitiven Bestimmung von Liganden, insbesondere solche höhermolekularer Spezifikation, unter Verwendung von Enzymmodulator als Marker des zu bestimmenden Liganden, von Enzymmodulator-Ligand-Konjugat bindendem Rezeptor, Enzym und Substrat, dadurch g e k e n n z e i c h n e t , dass mindestens einer der die Messreaktion beeinflussenden Reaktionspartner sterisch vergrössert wird.

2.      Verfahren nach Anspruch 1, dadurch g e - k e n n z e i c h n e t , dass die Vergrösserung durch Antikörper oder die entsprechenden Fab-Teile erfolgt.

3.      Verfahren nach Anspruch 1 oder 2, dadurch g e k e n n z e i c h n e t , dass als vergrösserte Reaktionskomponente

(a)      Anti-Rezeptor-Antikörper
(b)      Anti-Enzym-Antikörper
(c)      Anti-Enzymmodulator-Antikörper

oder eine geeignete Kombination hiervon verwendet werden.

4.      Verfahren nach einem oder mehreren der vorhergehenden Ansprüche, dadurch g e k e n n z e i c h n e t , dass die sterische Vergrösserung des Enzyms durch chemische Fixierung, vorzugsweise mittels
wasserlöslicher Polymerer, erfolgt.

5.    Verfahren nach einem oder mehreren der vorhergehenden Ansprüche, dadurch g e k e n n z e i c h -
n e t , dass die Reaktion unter Anwendung von an
sich bekannten Techniken durchgeführt wird.

6.    Verfahren nach einem oder mehreren der vorhergehenden Ansprüche, dadurch g e k e n n z e i c h -
n e t , dass als Marker ein das Enzym irreversibel
und/oder kovalent bindender Enzymmodulator eingesetzt
wird.

7.    Verfahren nach einem oder mehreren der vorhergehenden Ansprüche, dadurch g e k e n n z e i c h -
n e t , dass eine Kombination aus Anti-Rezeptor-
Antikörper mit Anti-Enzym-Antikörper bzw. chemisch
vergrössertem Enzym angewandt wird.

8.    Verfahren nach einem oder mehreren der vorhergehenden Ansprüche, dadurch g e k e n n z e i c h -
n e t , dass die sterische Vergrösserung des bzw.
der Reaktionspartner vor der Zusammenbringung der die
Messreaktion beeinflussenden Komponenten durchgeführt
wird.

- 1/2

Fig.1

Fig.2

Fig.3

Fig.4

- 2/2

## Fig.5a

## Fig.5b